# EUROPEAN PATENT APPLICATION

(11) **EP 4 378 937 A1**
(43) Date of publication of application: **05.06.2024**
(21) Application number: 22848706.2
(22) Date of filing: 29.07.2022
(51) Int. Cl.: C07D 401/14, A61P 35/00, A61P 35/02, A61K 31/506

(54) **INDOLE BIPYRIMIDINE COMPOUND, AND INTERMEDIATE THEREOF, PREPARATION METHOD THEREFOR AND USE THEREOF**

(30) Priority: 30.07.2021 CN 202110875626
(71) Applicant: Shanghai Allist Pharmaceuticals Co., Ltd., Shanghai 201318 (CN)
(72) Inventor: ZHANG, Qiang, Shanghai 201318 (CN)
(74) Representative: J A Kemp LLP
(86) International application number: PCT/CN2022/109170
(87) International publication number: WO 2023/006102

(57) **Abstract**

Disclosed in the present application are an indole bipyrimidine compound, and an intermediate thereof, a preparation method therefor and use thereof. The indole bipyrimidine compound, as shown in formula I, or a pharmaceutically acceptable salt thereof provided in the present invention has a good inhibitory effect on an EGFR Del19/T790M/C797S mutation, and is expected to treat and/or prevent a variety of diseases mediated by EGFR.

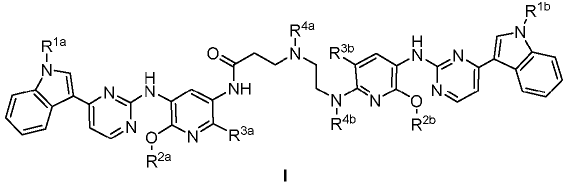

## Description

The present application claims the priority to Chinese patent application 2021108756267 filed on July 30, 2021. The full text of the above Chinese patent application is incorporated herein by reference.

### Technical Field

The present invention relates to an indole bipyrimidine compound, and an intermediate thereof, a preparation method therefor and use thereof.

### Background Art

Tumor is one of the major concerns that endanger human health, and lung cancer is one of the malignant tumors that pose the greatest threat to people's health and life. Lung cancer is mainly classified into small cell lung cancer (SCLC) and non-small cell lung cancer (NSCLC), with about 80% of lung cancer cases being NSCLC. The most common mutation seen in non-small cell lung cancer, and for which targeted drugs are existed, is the epidermal growth factor receptor (EGFR) mutation. Therefore, the use of epidermal growth factor receptor (EGFR) inhibitor, EGFR-TKI targeted drugs, is one of the most popular research hotspots for the treatment of lung cancer. EGFR (Epidermal Growth Factor Receptor) is a receptor for epidermal growth factor (EGF) cell proliferation and signaling. Studies have shown the presence of high or abnormal expression of EGFR in many solid tumors. EGFR is related to tumor cell's proliferation, angiogenesis, tumor invasion, metastasis and inhibition of apoptosis.

Currently, there are first, second and third generations of EGFR inhibitors on the market. The first-generation EGFR inhibitors are reversible targeted drugs, such as gefitinib, erlotinib, and icotinib. The second-generation EGFR inhibitors are irreversible targeted drugs, such as afatinib and dacomitinib. Although the first- and second- generation targeted drugs have remarkable efficacy, most patients will develop drug resistance within 1 to 2 years of using those drugs. Among patients with resistance to EGFR inhibitors, 50% of resistance is related to the T790M mutation. The third-generation EGFR-targeted drug osimertinib can bind to the T790M mutation site of EGFR-sensitive mutations and inhibit tumor resistance caused by the T790M mutation. Its advent has brought good survival benefits to more lung cancer patients. However, drug resistance for the third-generation targeted drugs will inevitably develop, and the reason for this resistance is the C797S mutation. The C797S mutation manifests itself in the mutation of a cysteine residue into serine. This mutation disrupts the binding of the EGFR protein to the third-generation targeted drugs, thereby failing to prevent the unilateral phosphorylation of EGFR and the activation of downstream signaling pathways. Currently, there are no mature treatments to address the following two triple mutations that are prone to occur after resistance to osimertinib: del19/T790M/C797S and L858R/T790M/C797S.

Therefore, it is of great research significance to target the C797S mutation, overcome resistance to osimertinib, and thus provide patients with safer and more effective EGFR inhibitors.

### Summary of the Invention

The technical problems to be solved by the present invention is to overcome the shortcomings of the existing EGFR inhibitors which have a single structure and poor inhibitory effect on C797S mutation, and provide an indole bipyrimidine compound and an intermediate thereof, a preparation method therefore and use thereof. The compound of the present invention has a novel structure, has good inhibitory effect on EGFR Del19/T790M/C797S mutation, and is expected to treat and/or prevent various diseases mediated by EGFR.

The present invention solves the above technical problems through the following technical solutions.

The present invention provides an indole bipyrimidine compound as shown in formula I or a pharmaceutically acceptable salt thereof, 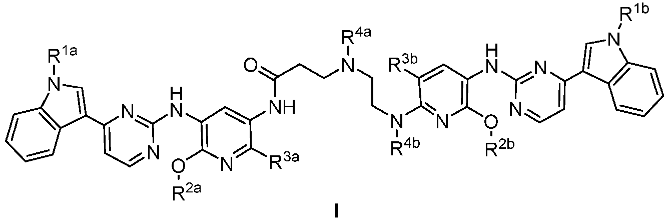
wherein, R^{1a} and R^{1b} are each independently H or C₁₋₄ alkyl;
R^{2a} and R^{2b} are each independently C₁₋₄ alkyl, and C₁₋₄ alkyl substituted by one or more R^{1-a}; when there is a plurality of substituents, they are the same or different;
R^{1-a} is independently halogen, -O-(C₁₋₄ alkyl), -N(R^{a1})(R^{a2}) or -C(=O)-(C₁₋₄ alkyl);
R^{a1} and R^{a2} are each independently H or C₁₋₄ alkyl;
R^{3a} and R^{3b} are each independently -N(R^{b1})(R^{b2});
R^{b1} and R^{b2} are each independently H, C₁₋₄ alkyl, -C₁₋₄ alkylene-N(R^{c1})(R^{c2}) or -C(=O)-R^{c3};
R^{c1}, R^{c2} and R^{c3} are each independently H, C₁₋₄ alkyl, C₁₋₄ alkyl substituted by one or more halogens, C₂₋₄ alkenyl, or C₂₋₄ alkenyl substituted by one or more halogens; and
R^{4a} and R^{4b} are each independently H or C₁₋₄ alkyl.

In certain preferred embodiments of the present invention, certain groups in the indole bipyrimidine compound as shown in formula I or a pharmaceutically acceptable salt thereof are defined as follows (groups not mentioned are as described in any embodiment of the present application, hereinafter referred to as "in a certain embodiment"):
when R^{1a} and R^{1b} are each independently C₁₋₄ alkyl, said C₁₋₄ alkyl is methyl, ethyl, n-propyl, isopropyl, n-butyl, isobutyl, sec-butyl or tert-butyl, for example, methyl or ethyl.

In a certain embodiment, when R^{2a} and R^{2b} are each independently C₁₋₄ alkyl and C₁₋₄ alkyl substituted by one or more R^{1-a}, C₁₋₄ alkyl in said C₁₋₄ alkyl and said C₁₋₄ alkyl substituted by the one or more R^{1-a} are methyl, ethyl, n-propyl, isopropyl, n-butyl, isobutyl, sec-butyl or tert-butyl, for example, methyl or ethyl.

In a certain embodiment, when there is a plurality of R^{1-a}, the number thereof is 2, 3, 4 or 5; for example, 2 or 3.

In a certain embodiment, when R^{1-a} is independently halogen, the halogen is independently fluoro, chloro, bromo or iodo; preferably fluoro or chloro.

In a certain embodiment, when R^{1-a} is independently -O-(C₁₋₄ alkyl) or -C(=O)-(C₁₋₄ alkyl), C₁₋₄ alkyl in said -O-(C₁₋₄ alkyl) and said -C(=O)-(C₁₋₄ alkyl) is methyl, ethyl, n-propyl, isopropyl, n-butyl, isobutyl, sec-butyl or tert-butyl, for example, methyl or ethyl.

In a certain embodiment, when R^{a1} and R^{a2} are each independently C₁₋₄ alkyl, said C₁₋₄ alkyl is methyl, ethyl, n-propyl, isopropyl, n-butyl, isobutyl, sec-butyl or tert-butyl, for example, methyl or ethyl.

In a certain embodiment, when R^{b1} and R^{b2} are each independently C₁₋₄ alkyl, the C₁₋₄ alkyl is methyl, ethyl, n-propyl, isopropyl, n-butyl, isobutyl, sec-butyl or tert-butyl, for example, methyl or ethyl.

In a certain embodiment, when R^{b1} and R^{b2} are each independently -C₁₋₄ alkylene-N(R^{c1})(R^{c2}), the C₁₋₄ alkylene is -CH₂-, -CH₂CH₂-, -CH(CH₃)-, -CH(CH₃)CH₂- or -C(CH₃)₂-, for example, -CH₂- or -CH₂CH₂-.

In a certain embodiment, when R^{c1}, R^{c2}, R^{c3}, R^{4a} and R^{4b} are each independently C₁₋₄ alkyl, said C₁₋₄ alkyl is methyl, ethyl, n-propyl, isopropyl, n-butyl, isobutyl, sec-butyl or tert-butyl, for example, methyl or ethyl.

In a certain embodiment, when R^{c1}, R^{c2} and R^{c3} are each independently C₁₋₄ alkyl substituted by one or more halogens, the C₁₋₄ alkyl is methyl, ethyl, n-propyl, isopropyl, n-butyl, isobutyl, sec-butyl or tert-butyl, for example, methyl or ethyl.

In a certain embodiment, when R^{c1}, R^{c2} and R^{c3} are each independently C₂₋₄ alkenyl or C₂₋₄ alkenyl substituted by one or more halogens, C₂₋₄ alkenyl in said C₂₋₄ alkenyl and said C₂₋₄ alkenyl substituted by the one or more halogens is ethenyl, propenyl or allyl, for example, propenyl or allyl.

In a certain embodiment, when ^{2a} and R^{2b} are each independently C₁₋₄ alkyl substituted by one or more R^{1-a} and R^{1-a} is halogen, the C₁₋₄ alkyl substituted by R^{1-a} is trifluoromethyl or -CH₂CF₃.

In a certain embodiment, when R^{c1}, R^{c2} and R^{c3} are each independently C₁₋₄ alkyl substituted by one or more halogens, the C₁₋₄ alkyl substituted by halogens is -CH₂CH₂Cl or -CH₂CF₃.

In a certain embodiment, R^{b1} and R^{b2} are each independently selected from H, methyl, 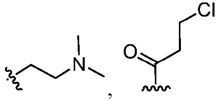 and

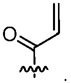

In a certain embodiment, one of R^{b1} and R^{b2} is C₁₋₄ alkyl, and the other is -C₁₋₄ alkylene-N(R^{c1})(R^{c2}).

In a certain embodiment, one of R^{b1} and R^{b2} is H, and the other is H or -C(=O)-R^{c3}.

In a certain embodiment, -N(R^{b1})(Rb²) is independently selected from NH₂, 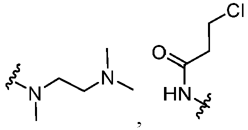 and

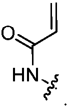

In a certain embodiment, one of R^{3a} and R^{3b} is 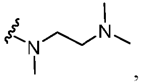 and the other is NH₂, 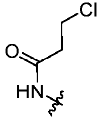 or

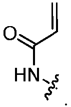

In a certain embodiment, the indole bipyrimidine compound as shown in formula I can be the following compound:

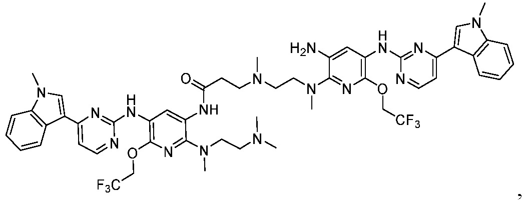

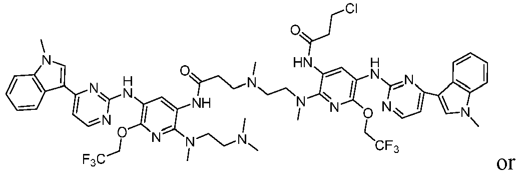

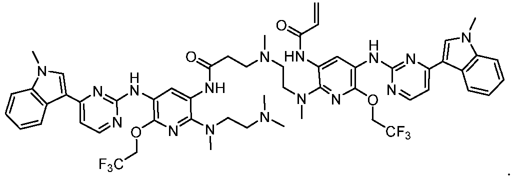

In a certain embodiment, the pharmaceutically acceptable salt of the indole bipyrimidine compound as shown in formula I can be the following compound:

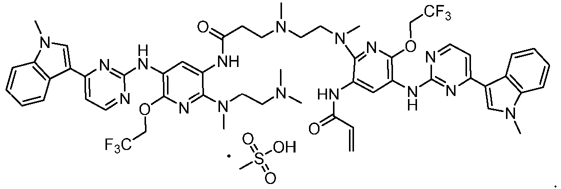

In the present invention, the indole bipyrimidine compound as shown in formula I or a pharmaceutically acceptable salt thereof can be synthesized by a method including those similar to a well-known method in the chemical field. The steps and conditions of said method can refer to the steps and conditions for a similar reaction in the field, in particular, the synthesis is carried out according to the description herein. Starting materials are generally obtained from commercial sources such as Aldrich or can be readily prepared using methods well known to those skilled in the art (available through SciFinder and Reaxys online databases).

In the present invention, for the indole bipyrimidine compound as shown in formula I or a pharmaceutically acceptable salt thereof, other indolobipyrimidine compounds as shown in formula I or a pharmaceutically acceptable salt thereof may further be prepared by using conventional methods in the art to peripherally modify the obtained indole bipyrimidine compound as shown in formula I or a pharmaceutically acceptable salt thereof.

Necessary raw materials or reagents for preparing the indole bipyrimidine compounds as shown in formula I or a pharmaceutically acceptable salt thereof may be commercially available, or prepared by synthetic methods known in the art. The compounds of the present invention may be prepared as free bases or as salts formed by adding acid to them by using methods as described in the experimental section below. The term "pharmaceutically acceptable salt" refers to a pharmaceutically acceptable salt as defined herein and has all the effects of the parent compound.

The present invention further provides a preparation method for an indole bipyrimidine compound as shown in formula I or a pharmaceutically acceptable salt thereof, which can be of the following routes:
route I
when R^{3b} is -NH₂, the corresponding indole bipyrimidine compound as shown in formula I is as shown in formula I-1, which can be prepared by the following method: 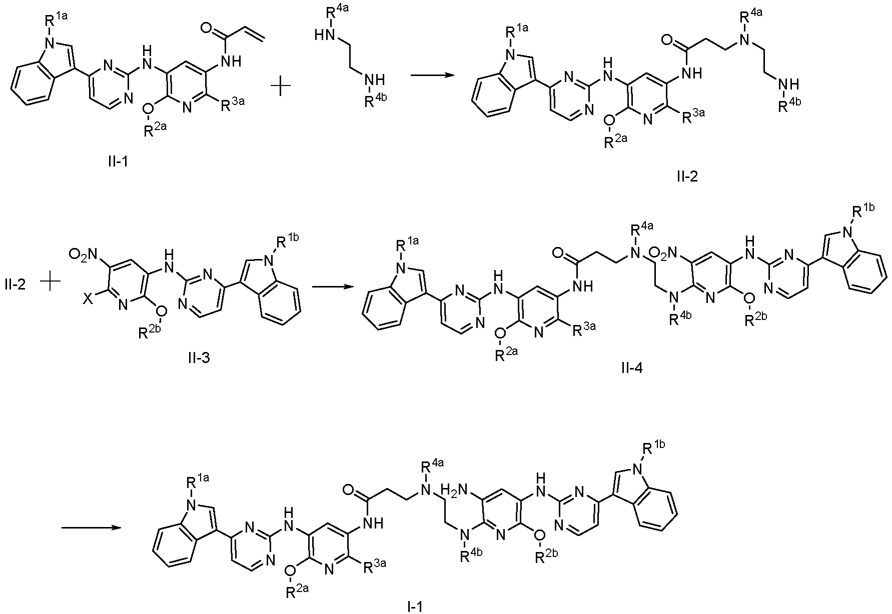 wherein, R^{1a}, R^{1b}, R^{2a}, R^{2b}, R^{3a}, R^{4a} and R^{4b} are as defined in any one of the above embodiments; X is a leaving group, such as halogen, also such as Cl;
step 1: in a solvent and in the presence of a base, a compound as shown in formula II-1 and a compound as shown in formula (R^{4a}H)NCH₂CH₂N(HR^{4b}) are subjected to an addition reaction as shown to afford a compound as shown in formula II-2;
the operations and conditions for the addition reaction and post-treatment thereof may be conventional operations and conditions for this type of reaction in the art; for example, said base may be an organic base, such as triethylamine, N,N-diisopropylethylamine or N,N'-dimethylethylenediamine, also such as triethylamine; said solvent may be tetrahydrofuran, and the reaction may be heating until the system refluxes; a molar ratio of said compound as shown in formula II-1 to the compound as shown in formula (R^{4a}H)NCH₂CH₂N(HR^{4b}) may be 1:5; a molar ratio of said compound as shown in formula II-1 to the solvent is 1:2; a mass-to-volume ratio of said compound as shown in formula II-1 to the solvent may be 0.1 Kg/L to 0.2 Kg/L, such as 0.1 Kg/L;
step 2: in a solvent and in the presence of a base, the compound as shown in formula II-2 and a compound as shown in formula II-3 or a salt thereof are subjected to a substitution reaction as shown to afford a compound as shown in formula II-4; and
the operations and conditions for the substitution reaction and post-treatment thereof may be conventional operations and conditions for this type of reaction in the art; for example, said base may be an inorganic base, such as potassium carbonate; said solvent may be DMF or DMSO (such as DMF), and the temperature of the reaction may be 30°C to 80°C (such as 65°C); said salt of the compound as shown in formula II-3 may be a p-toluenesulfonate of said compound; a molar ratio of the compound as shown in formula II-2 to the compound as shown in formula II-3 or a salt thereof may be 0.9:1 to 1:0.9, such as 1:0.95; a molar ratio of the compound as shown in formula II-2 to the base may be 1:3; a mass-to-volume ratio of the compound as shown in formula II-2 to the solvent may be 0.1 Kg/L to 0.2 Kg/L, such as 0.144 Kg/L;
step 3: in a solvent and in the presence of a reducing agent, the compound as shown in formula II-4 is subjected to a nitro reduction reaction as shown, and optionally, an acid is added to promote the completion of the reduction reaction, so as to afford the compound as shown in formula I-1;
the operations and conditions for the nitro reduction reaction and post-treatment thereof may be conventional operations and conditions for this type of reaction in the art; for example, said reducing agent may be sodium dithionite, iron powder, zinc powder or H₂, such as sodium dithionite, wherein the reduction of the compound as shown in formula II-4 with H₂ is carried out in the presence of a catalyst such as Pd/C and Pd(OH)₂/C; said solvent may be tetrahydrofuran and water or may be ethanol and water (such as tetrahydrofuran and water), and the temperature of the reaction may be 25°C to 50°C (for example, 35°C-45°C); a molar ratio of the compound as shown in formula II-4 to said reducing agent may be 1:6; said acid may be a concentrated hydrochloric acid; a molar ratio of the compound as shown in formula II-4 to said acid may be 1:44; a mass-to-volume ratio of the compound as shown in formula II-4 to the solvent may be 0.05Kg/L to 0.1 Kg/L, such as 0.05 Kg/L;
route II
when R^{3b} is -NH-C(=O)-R^{c3}, the corresponding indole bipyrimidine compound as shown in formula I is as shown in formula I-2, which can be prepared by the following method: 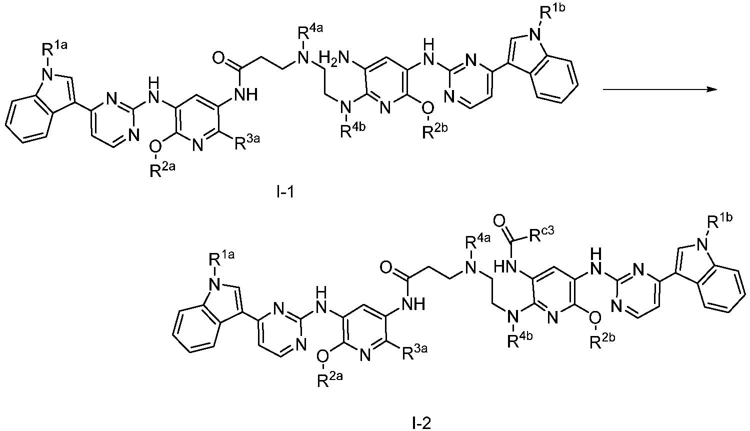
wherein, R^{1a}, R^{1b}, R^{2a}, R^{2b}, R^{3a}, R^{c3}, R^{4a} and R^{4b} are as defined in any one of the above embodiments;
in a solvent and in the presence of a base, the compound as shown in formula I-1 and a compound as shown in formula Cl-C(=O)-R^{c3} are subjected to a substitution reaction as shown to afford a compound as shown in formula I-2;
the operations and conditions for the substitution reaction and post-treatment thereof may be conventional operations and conditions for this type of reaction in the art; for example, said base may be an organic base, such as triethylamine or N,N-diisopropylethylamine, also such as triethylamine; said solvent may be dichloromethane or tetrahydrofuran (such as dichloromethane), and the temperature of the reaction may be -50°C to 10°C (such as -40°C); a molar ratio of the compound as shown in formula I-1 to a compound as shown in formula Cl-C(=O)-R^{c3} may be 1:1.5; a molar ratio of the compound as shown in formula 1-1 to the base may be is 1:2; a mass-to-volume ratio of the compound as shown in formula I-1 to the solvent may be 0.05 Kg/L to 0.15 Kg/L, such as 0.1 Kg/L;
route III
when R^{3b} is -NH-C(=O)-CH=CH₂, the corresponding indole bipyrimidine compound as shown in formula I is as shown in formula I-2b, which can be prepared by the following method: 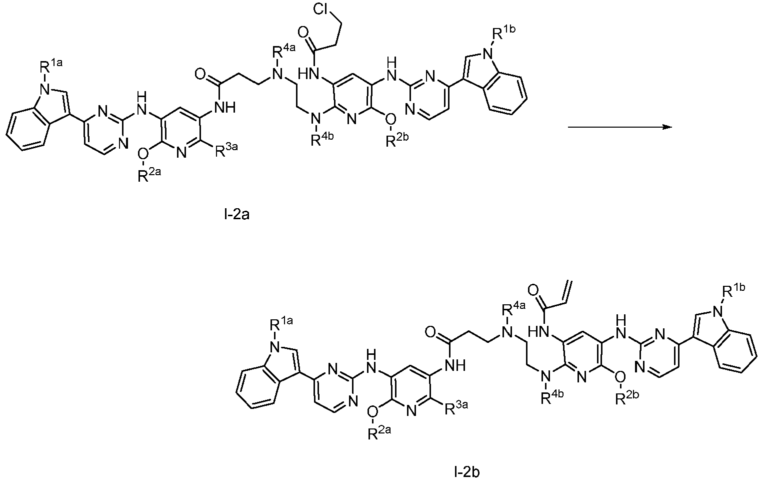
wherein, R^{1a}, R^{1b}, R^{2a}, R^{2b}, R^{3a}, R^{4a} and R^{4b} are as defined in any one of the above embodiments;
in a solvent and in the presence of a base, a compound as shown in formula I-2a is subjected to an elimination reaction as shown to afford a compound as shown in formula I-2b;
the operations and conditions for the elimination reaction and post-treatment thereof may be conventional operations and conditions for this type of reaction in the art; for example, said base can be an organic base, such as triethylamine or N,N-diisopropylethylamine, also such as triethylamine; said solvent may be acetonitrile or DMSO (such as acetonitrile), and the temperature of the reaction may be 50°C to 100°C (such as 80°C); a molar ratio of said compound as shown in formula I-2a to the base may be 1:5; and a mass-to-volume ratio of the compound as shown in formula I-2a to the solvent may be 0.05 Kg/L to 0.15 Kg/L, such as 0.1 Kg/L.

The present invention further provides a compound as shown below,

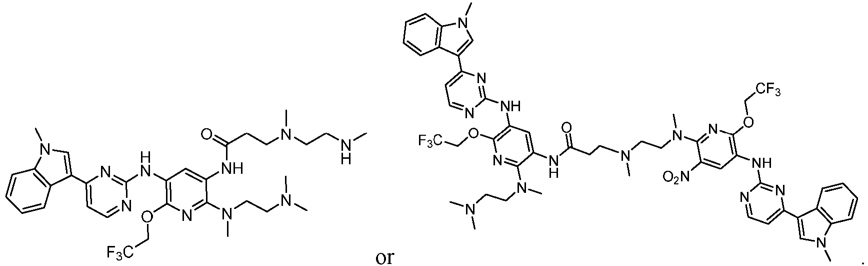

The present invention further provides a pharmaceutical composition, wherein the pharmaceutical composition comprises a therapeutically effective amount of substance A and pharmaceutical auxiliary materials (or pharmaceutically acceptable carriers); and the substance A is the above indole bipyrimidine compound as shown in formula I or a pharmaceutically acceptable salt thereof.

The present invention further provides use of a substance A in the preparation of a EGFR inhibitor, wherein the substance A is the above indole bipyrimidine compound as shown in formula I or a pharmaceutically acceptable salt thereof, and said EGFR comprises an EGFR Del19/T790M/C797S mutation. In said use, said EGFR inhibitor may be used in vivo of a mammalian organism; and may also be used in vitro, mainly for experimental purposes, for example: used as a standard sample or control sample to provide a comparison, or prepared into a kit according to conventional methods in the art to provide rapid detection of the effect of inhibiting EGFR.

The present invention further provides use of a substance A in the preparation of a medicament, wherein said medicament is for use in the treatment and/or prevention of a disease mediated by EGFR (an activating or drug-resistant mutant); said substance A is the above indole bipyrimidine compound as shown in formula I or a pharmaceutically acceptable salt thereof; and said substance A is at a therapeutically effective amount.

The present invention further provides use of a substance A in the preparation of a medicament, wherein said medicament is for use in the treatment and/or prevention of cancer; said substance A is the above indole bipyrimidine compound as shown in formula I or a pharmaceutically acceptable salt thereof; and said substance A is at a therapeutically effective amount.

The present invention further provides a method for inhibiting EGFR (an activating or drug-resistant mutant), wherein said method comprises administering a therapeutically effective amount of substance A to a patient; and said substance A is the above indole bipyrimidine compound as shown in formula I or a pharmaceutically acceptable salt thereof.

The present invention further provides a method for treating and/or preventing a disease mediated by EGFR (an activating or drug-resistant mutant), wherein said method comprises administering a therapeutically effective amount of substance A to a patient; and said substance A is the above indole bipyrimidine compound as shown in formula I or a pharmaceutically acceptable salt thereof.

The present invention further provides a method for treating and/or preventing cancer, where in sad method comprises administering a therapeutically effective amount of substance A to a patient; and said substance A is the above indole bipyrimidine compound as shown in formula I or a pharmaceutically acceptable salt thereof.

The disease mediated by EGFR as described above is a disease resistant to first-, second- and third- generation EGFR inhibitors; and the first-, second- and third- generation EGFR inhibitors may be selected from gefitinib, erlotinib, icotinib, afatinib, dacomitinib and osimertinib.

The disease mediated by EGFR as described above may be a disease mediated by an EGFR Del19/T790M/C797S mutation.

The disease mediated by EGFR as described above may be cancer.

The cancer as described above may be selected from one or more of colon cancer, pancreatic cancer, breast cancer, prostate cancer, lung cancer, brain cancer, ovarian cancer, cervical cancer, testicular cancer, renal cancer, cancer of the head or neck, bone cancer, skin cancer, rectal cancer, liver cancer, colorectal cancer, non-small cell lung cancer, small cell lung cancer, adenocarcinoma of the lung, squamous carcinoma of the lung, esophageal cancer, gastric cancer, thyroid cancer, bladder cancer, lymphoma, glioma, glioblastoma, gastrointestinal stromal tumor, cholangiocarcinoma, endometrial cancer, multiple myeloma, leukemia and melanoma.

In addition to the foregoing, when used in the specification and claims of the present application, unless otherwise specified, the following terms have the meanings shown below.

The term "a plurality of' means 2, 3, 4 or 5.

The term "pharmaceutically acceptable salt" refers to a salt prepared from compounds of the present invention and relatively non-toxic, and pharmaceutically acceptable acids or bases. When compounds of the present invention contain relatively acidic functional groups, base addition salts may be obtained in a manner of contacting the neutral form of such compounds with a sufficient amount of a pharmaceutically acceptable base in a pure solution or a suitable inert solvent. Pharmaceutically acceptable base addition salts include, but are not limited to: lithium salts, sodium salts, potassium salts, calcium salts, aluminum salts, magnesium salts, zinc salts, bismuth salts, ammonium salts, and diethanolamine salts. When compounds of the present invention contain relatively basic functional groups, acid addition salts may be obtained by contacting the neutral form of such compounds with a sufficient amount of a pharmaceutically acceptable acid in pure solution or a suitable inert solvent. Said pharmaceutically acceptable acid comprises inorganic acid and organic acid (such as methanesulfonic acid). For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

As used herein, the compounds as shown in formula I of the present invention may contain one or more chiral centers and exist in different optically active forms. A compound contains enantiomers when it contains a chiral center. The present invention includes these two isomers and mixtures of isomers, such as racemic mixtures. Enantiomers may be resolved by methods known in the art, such as a crystallization method and a chiral chromatography method. When a compound of formula I contains more than one chiral center, diastereoisomers may exist. The present invention includes resolved optically pure specific isomers as well as mixtures of diastereoisomers. Diastereoisomers may be resolved by methods known in the art, such as crystallization and preparative chromatography. The term "stereoisomer" includes conformational isomers and configurational isomers, where configurational isomers mainly include cis-trans isomers and optical isomers. The compounds described in the present invention may be present in the form of stereoisomers, and therefore encompass all possible stereoisomer forms, including, but not limited to, cis-trans isomers, enantiomers, diastereoisomers, atropisomers, etc., and the compounds described in the present invention may be also present in the form of any combination or any mixture of the above stereoisomers, such as equal mixtures of mesomers, racemates, atropisomers, etc., for example, a single enantiomer, a single diastereoisomer or a mixture thereof, or a single atropisomer or a mixture thereof. When the compound described in the present invention contains an olefin double bond, unless otherwise stated, it includes cis isomers and trans isomers, as well as any combination thereof. The term "single stereoisomer" means that the mass content of one stereoisomer of the compound of the present invention relative to all stereoisomers of the compound is not less than 95%. The compound of formula I has optical isomers derived from asymmetric carbon, axial asymmetry, etc. If necessary, a single isomer may be obtained by resolving according to methods known in the art, such as a crystallization method or a chromatography method (such as chiral chromatography).

As described above, the present invention provides compounds as shown in the above structures, or cis-trans isomers, mesomers, racemates, enantiomers, diastereoisomers, atropisomers or mixtures thereof, wherein "mixtures thereof' include any form of mixing between any one of the aforementioned stereoisomers (such as cis-trans isomers, enantiomers, diastereoisomers, and atropisomers) and/or mixtures (mesomers and racemate), such as mixing of mixtures of cis-trans isomers, mixtures of enantiomers and diastereoisomers, mixtures of diastereoisomers, mixtures of atropisomers, or mixtures of cis-trans isomers and racemates, mixing of mixtures of enantiomers and diastereoisomers, mixing of mixtures of atropisomers and diastereoisomer, etc.

The indole bipyrimidine compound as shown in formula I of the present invention or a pharmaceutically acceptable salt thereof may contain unnatural proportions of atomic isotopes on one or more atoms constituting the compound. For example, the compound may be labeled with radioactive isotopes, such as deuterium (D), tritium (³H), iodine-125 (¹²⁵I) or C-14 (¹⁴C). All variations in the isotopic composition of the compounds of the present invention, whether radioactive or not, are included within the scope of the present invention.

In the present application, "pharmaceutical composition" refers to a formulation containing a compound of the present invention and a medium generally accepted in the art for delivering the biologically active compound to a mammal, such as a human. The medium includes a pharmaceutically acceptable carrier. The purpose of a pharmaceutical composition is to facilitate administration to organisms and facilitate the absorption of active ingredients to exert biological activity.

In the present application, "pharmaceutically acceptable" refers to a substance (such as pharmaceutical auxiliary materials) that does not affect the biological activity or properties of the compounds of the present invention and is relatively non-toxic, that is, the substance may be administered to individuals without causing adverse biological reaction or interact in an adverse manner with any component contained in the composition.

The term "pharmaceutical auxiliary materials" or "pharmaceutically acceptable carriers" refers to excipients and additives used in the production of pharmaceuticals and formulation of prescriptions, and they are all substances other than active ingredients included in pharmaceutical preparations. Please refer to the Pharmacopoeia of the People's Republic of China (2015 Edition), Part IV, or Handbook of Pharmaceutical Excipients (Raymond C Rowe, 2009 Sixth Edition). Auxiliary materials are mainly for use in providing a safe, stable and functional pharmaceutical composition, and may also provide a method to enable the active ingredients to dissolve at a desired rate after administration to a subject, or to promote the effective absorption of the active ingredient after administration of the composition to the subject. Said pharmaceutical auxiliary materials may be inert fillers, or provide certain functions, such as stabilizing the overall pH value of the composition or preventing degradation of the active ingredients of the composition. Said pharmaceutical auxiliary materials may include one or more of the following auxiliary materials: binders, suspending agents, emulsifiers, diluents, fillers, granulating agents, adhesives, disintegrants, lubricants, antiadhesion agents, glidants, wetting agents, gelling agents, absorption delaying agents, dissolution inhibitors, enhancers, adsorbents, buffers, chelating agents, preservatives, colorants, flavorings and sweeteners.

Pharmaceutical compositions of the present invention may be prepared according to the disclosure using any method known to those skilled in the art. For example, conventional mixing, dissolving, granulating, emulsifying, grinding, encapsulating, embedding or freezedrying processes.

When used as a medicament, the indole bipyrimidine compound as shown in formula I or a pharmaceutically acceptable salt thereof may be administered in any form of a pharmaceutical composition. These compositions may be prepared according to methods well known in the art of pharmacy and may be administered by a variety of routes, depending on the desired local or systemic treatment and the area to be treated. Administration may be topical (including epidermal and transdermal, ocular and mucosal, including intranasal, vaginal and rectal delivery), pulmonary (e.g., by inhalation or insufflation of powder or aerosol, including by a nebulizer; intratracheal or intranasal), oral (solid and liquid formulations) or parenteral forms of administration. Examples of solid oral formulations include, but are not limited to, powders, capsules, caplets, softgels, and tablets. Examples of liquid formulations for oral or mucosal administration include, but are not limited to, suspensions, emulsions, elixirs, and solutions. Examples of topical formulations include, but are not limited to, emulsions, gels, ointments, creams, patches, pastes, foams, lotions, drops, or serum formulations. Examples of formulations for parenteral administration include, but are not limited to, injectable solutions, dry formulations which may be dissolved or suspended in a pharmaceutically acceptable carrier, injectable suspensions, and injectable emulsions. Pharmaceutical compositions and formulations for topical administration may include transdermal patches, salves, lotions, ointments, gels, drops, suppositories, sprays, liquids and powders. Examples of other suitable formulations of said pharmaceutical composition include, but are not limited to, eye drops and other ophthalmic formulations; and aerosols, such as nasal sprays or inhalants. Oral administration may include dosage forms formulated for once a day or bis in die (BID) administration. Parenteral administration includes intravenous, intraarterial, subcutaneous, intraperitoneal, intramuscular, or injection or infusion administration; or intracranial (such as intrathecal) or intraventricular administration. Parenteral administration may be in the form of a single bolus dose, or may be carried out by a continuous infusion pump. Conventional pharmaceutical carriers, aqueous, powdered or oily bases, thickening agents and the like may be necessary or desirable. Pharmaceutical compositions including the present invention may also be in controlled or delayed release dosage forms (for example, liposomes or microspheres).

The term "treatment" refers to therapeutic therapy or palliative measures. When referring to a specific condition, treatment means: (1) alleviating the condition or one or more biological manifestations of the condition, (2) interfering with (a) one or more points in the biological cascade that results in or causes the condition or (b) one or more biological manifestations of the condition, (3) improving one or more symptoms, effects, or side effects associated with the condition, or one or more symptoms, effects, or side effects associated with the condition or its treatment, or (4) slowing the progression of the condition or one or more biological manifestations of the condition. "Treatment" may also refer to prolonging survival as compared to expected survival without treatment.

The term "prevention" refers to reducing the risk of acquiring or developing a disease or disorder.

The term "therapeutically effective amount" refers to an amount of a compound sufficient to effectively treat the disease or condition described herein when administered to a patient. The "therapeutically effective amount" will vary depending on the compound, the condition and its severity, and the age of the patient to be treated, but can be adjusted as necessary by those skilled in the art.

The term "patient" refers to any animal, preferably a mammal, and most preferably a human, to which the compound or composition is or has been administered in accordance with embodiments of the present invention. The term "mammal" includes any mammal. Examples of mammals include, but are not limited to, cattle, horses, sheep, pigs, cats, dogs, mice, rats, rabbits, guinea pigs, monkeys, humans, etc., with humans being the most preferred.

Unless otherwise stated, the following definitions as used herein shall be applied. For the purposes of the present invention, chemical elements are in accordance with the CAS version of the Periodic Table, and Handbook of Chemistry and Physics, 75th Edition, 1994. In addition, general principles of organic chemistry may be referred to the description in "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are incorporated herein by reference.

In the present specification, groups and their substituents may be selected by those skilled in the art to provide stable structural moieties and compounds. When a substituent is described by a conventional chemical formula written from left to right, the substituent also includes chemically equivalent substituents obtained when the structural formula is written from right to left.

Certain chemical groups defined herein are preceded by a shorthand notation to represent the total number of carbon atoms present in the group. For example, C₁-C₄ alkyl or C₁₋₄alkyl refers to alkyl as defined below having a total of 1, 2, 3 or 4 carbon atoms. The total number of carbon atoms in the shorthand notation does not include carbons that may be present in substituents of the group.

As used herein, the numerical range defined in the substituent such as 0 to 4, 1-4, 1 to 3, etc. indicates the integer within the range, such as 1-6 indicating 1, 2, 3, 4, 5, or 6.

The term "comprising" is an open-ended expression, that is, it includes the contents indicated in the present invention, but does not exclude other contents.

The term "substituted" means that any one or more hydrogen atoms on a specified atom are substituted by a substituent, as long as the valence state of the specified atom is normal and the substituted compound is stable.

In general, the term "substituted" means that one or more hydrogen atoms in a given structure are substituted by a specified substituent. Further, when the group is substituted by more than one substituent, said substituents are independent of each other, that is, the more than one substituent may be different from each other or may be the same. Unless otherwise indicated, a substituent group may be substituted at each substitutable position of the substituted group. When more than one position in a given structural formula can be substituted by one or more substituents selected from a specific group, the substituents may be identically or differently substituted at each position.

In various parts of the present specification, substituents of the compounds disclosed in the present invention are disclosed according to group types or ranges. In particular, the present invention includes each individual sub-combination of individual members of these group types and ranges. The term "Cₓ-C_{y} alkyl" or "C_{x-y} alkyl" refers to a linear or branched saturated hydrocarbon containing x to y carbon atoms. For example, the term "C₁-C₆ alkyl" or "C₁₋₆alkyl" particularly refers to independently disclosed methyl, ethyl, C₃ alkyl, C₄ alkyl, C₅ alkyl and C₆ alkyl; and "C₁₋₄ alkyl" particularly refers to independently disclosed methyl, ethyl, C₃ alkyl (i.e. propyl, including n-propyl and isopropyl), and C₄ alkyl (i.e. butyl, including n-butyl, isobutyl, sec-butyl and tert-butyl).

As used herein, the terms "moiety," "structural moiety," "chemical moiety," "group," and "chemical group" refer to a specific fragment or functional group in a molecule. Chemical moieties are generally thought of as chemical entities embedded in or attached to the molecule.

When an enumerated substituent does not indicate the atom through which it is connected into the general chemical structure (including but not specifically mentioned compounds), such substituent may be bonded through any atom thereof. Combinations of substituents and/or variants thereof are permitted only if such combinations result in stable compounds.

When any variable (for example, R^{1-a}) appears multiple times in the definition of a compound, the definition of the variable appearing at each position has nothing to do with the definitions of the variable appearing at the remaining positions. Their meanings are independent of each other and do not affect each other. Therefore, if a group is substituted by 1, 2 or 3 R^{1-a} groups, that is to say, the group may be substituted by up to 3 R^{1-a}, where the definition of R^{1-a} at a certain position is independent of the definition of R^{1-a} at the remaining positions. Additionally, combinations of substituents and/or variables are permitted only if such combinations result in stable compounds.

When an enumerated group does not expressly indicate that it has a substituent, such group only indicates to being unsubstituted. For example, when "C₁₋₄ alkyl" is not preceded by the definition of "substituted or unsubstituted", it only refers to "C₁₋₄ alkyl" itself or "unsubstituted C₁₋₄ alkyl".

In various parts of the present invention, linking substituents are described. When the structure clearly requires a linking group, Markush variables enumerated for the group should be understood as referring to the linking group. For example, if the structure requires a linking group and the Markush group definition for the variable enumerates "alkyl", it will be understood that the "alkyl" represents a linked alkylene group.

In some specific structures, when an alkyl group is expressly represented as a linking group, the alkyl group represents a linked alkylene group, and for example, C₁₋₄ alkyl in the group "halo-C₁₋₄ alkyl" should be understood as C₁₋₄ alkylene.

The term "halogen" refers to fluoro, chloro, bromo or iodo, especially F or Cl.

In the present application, the term "alkyl", as a group itself or part of another group (for example, as used in haloalkyl, deuterated alkyl, etc.), refers to a branched and linear saturated aliphatic hydrocarbon group including and having a specified number of carbon atoms, consisting only of carbon atoms and hydrogen atoms, having, for example, 1 to 12 (preferably 1 to 8, more preferably 1 to 6, more preferably 1 to 4) carbon atoms, and connected to the rest of a molecule through a single bond, wherein propyl is C₃ alkyl (including isomers, for example, n-propyl or isopropyl); butyl is C₄ alkyl (including isomers, for example, n-butyl, sec-butyl, isobutyl or tert-butyl); pentyl is C₅ alkyl (including isomers, for example, n-pentyl, 1-methyl-butyl, 1-ethyl-propyl, 2-methyl-1-butyl, 3-methyl-1-butyl, isopentyl, tert-pentyl or neopentyl); hexyl is C₆ alkyl (including isomers, for example, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, and 2,3-dimethylbutyl). Examples include, but are not limited to, methyl, ethyl, n-propyl, isopropyl, n-butyl, isobutyl, sec-butyl, tert-butyl, n-pentyl, 2-methylbutyl, 2,2-dimethylpropyl, n-hexyl, n-heptyl, 2-methylhexyl, 3-methylhexyl, n-octyl, nonyl and decyl and other similar alkyls.

In the present application, the term "alkylene", as a group itself or part of another group, refers to a saturated divalent hydrocarbon group obtained by removing two hydrogen atoms from a saturated linear or branched hydrocarbon group; that is, one of the hydrogens in the alkyl is substituted, alkyl being as defined above. Examples of alkylene groups include methylene (-CH₂-), ethylidene{including -CH₂CH₂- or -CH(CH₃)-}, isopropylidene {including -CH(CH₃)CH₂- or -C(CH₃)₂-} and so on.

In the present application, the term "alkenyl", as a group itself or part of another group, refers to a linear or branched hydrocarbon chain group having at least one double bond, consisting only of carbon atoms and hydrogen atoms, having for example, 2 to 12 (preferably 2 to 8, more preferably 2 to 6, and most preferably 2 to 4) carbon atoms, and connected to the rest of a molecule through a single bond, for example, including, but not limited to, ethenyl, n-propenyl, isopropenyl, n-butenyl, isobutenyl, sec-butenyl, tert-butenyl, n-pentenyl, 2-methylbutenyl, 2,2-dimethylpropenyl, n-hexenyl, heptenyl, 2-methylhexenyl, 3-methylhexenyl, octenyl, nonenyl and decenel, etc.

It should be understood that, as used in the present invention, singular forms, such as "a" and "an", include plural referents, unless otherwise specified.

The term "one or more" or "one or more than two" means 1, 2, 3, 4, 5, 6, 7, 8, 9 or more.

Unless otherwise stated, the present invention employs traditional methods such as mass spectrometry and elemental analysis, and each step and condition can refer to the conventional operating steps and conditions in the art.

Unless otherwise indicated, the present invention employs standard nomenclature and standard laboratory procedures and techniques of analytical chemistry, organic synthetic chemistry, and optics. In some cases, standard techniques are used for chemical synthesis, chemical analysis, and performance testing of light-emitting devices.

In addition, it should be noted that, unless otherwise clearly stated, the manner of description "is independently" or "are independently" used in the present invention should be understood in a broad sense, meaning that each individual described is independent of each other and can independently be the same or different specific groups. In more detail, the manner of description "is independently" or "are independently" can mean that in different groups, the specific options expressed between the same symbols do not affect each other, or that in the same group, the specific options expressed between the same symbols do not affect each other.

Those skilled in the art can understand that according to the conventions used in the art, " 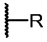 " and " 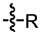 " used in the structural formulas of the groups described in the present application means that a corresponding group R is attached to other segments and groups in the compound through said site.

Those skilled in the art can understand that according to the conventions used in the art, "-̅ -̅ -̅" used in the structural formulas of groups described in the present application represents a single bond or a double bond.

Unless otherwise specified, all technical and scientific terms used herein have their standard meaning in the art to which the claimed subject matter pertains. If there isa plurality of definitions for a term, the definition herein shall prevail.

On the basis of not violating common sense in the art, the above preferred conditions can be combined arbitrarily to obtain preferred examples of the present invention.

The reagents and raw materials used in the present invention are all commercially available.

The positive and progressive effect of the present invention is that the indole bipyrimidine compound has a good inhibitory effect on an EGFR Del19/T790M/C797S mutation, and is expected to treat and/or prevent a variety of diseases mediated by EGFR, for example, diseases resistant to first-, second- and third- generation EGFR inhibitors, and diseases mediated by the EGFR Del19/T790M/C797S mutation.

### Detailed Description of Embodiments

The present invention is further described below by means of examples, but the present invention is not limited to the scope of the described examples. Experimental methods for which specific conditions are not indicated in the following examples should be selected according to conventional methods and conditions, or according to product specifications.

In the present invention, the room temperature refers to the ambient temperature, which is 10°C-35°C. Overnight refers to 8-15 hours. Reflux refers to the solvent reflux temperature under normal pressure.

### Example 1

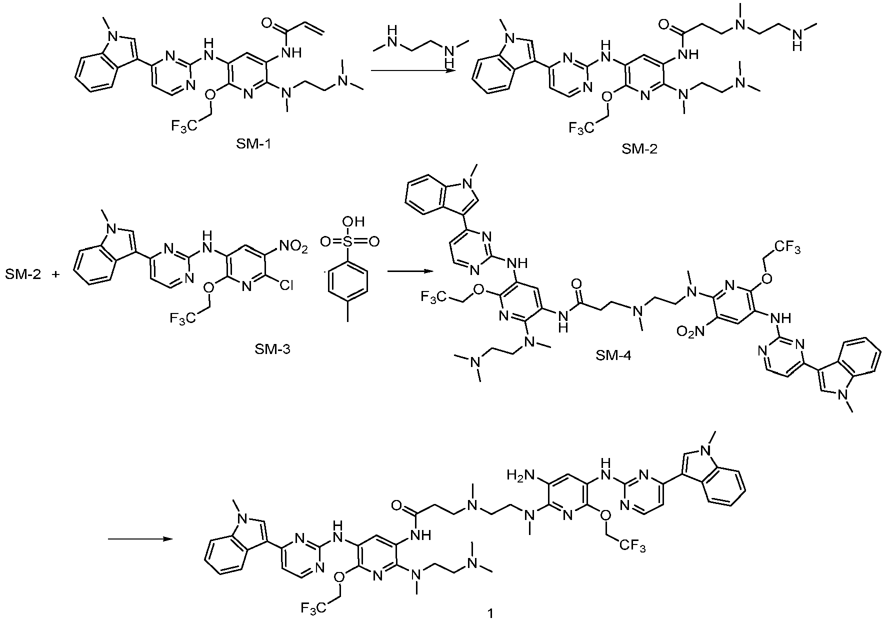

### Preparation of SM-2

Add SM-1 (300 g, 1.0 eq.), N,N-dimethylethylenediamine (232 g, 5.0 eq.), and triethylamine (107 g, 2.0 eq.) into a four- necked flask, add tetrahydrofuran (3 L), heat until the system refluxes for 24 hours, stop heating when the raw material reaction is complete, and concentrate under reduced pressure to afford a 346 g of crude product with a purity of 97.7% and a yield of 100%.
MS m/z: 657.5 [M+H]⁺;
¹H NMR (400 MHz, DMSO-d6) δ 9.97 - 9.86 (m, 1H), 8.43 (d, J = 14.9 Hz, 2H), 8.27 (d, J = 5.4 Hz, 2H), 8.18 (s, 1H), 7.51 (t, J = 7.6 Hz, 1H), 7.25 (t, J = 7.5 Hz, 1H), 7.16 (dd, J = 14.7, 6.3 Hz, 2H), 4.97 (q, J = 9.1 Hz, 2H), 3.32 - 3.25 (m, 2H), 3.20 (dd, J = 13.3, 6.1 Hz, 2H), 2.95 - 2.77 (m, 5H), 2.68 (dt, J = 13.3, 6.9 Hz, 3H), 2.57 (t, J = 6.1 Hz, 3H), 2.44 (d, J = 3.3 Hz, 6H), 2.29 - 2.17 (m, 10H), 2.12 (d, J = 6.1 Hz, 1H).

### Preparation of SM-4

Add **SM-2** (346 g, 1.0 eq.), **SM-3** (325 g, 0.95 eq.), and potassium carbonate (218 g, 3.0 eq.) to a four-necked flask, add DMF (2.4 L), heat to 65°C, allow to react for 8 hours while holding this temperature, monitor until the raw material reaction is complete, stop heating, add the reaction system to water (12 L), stir, carry out suction filtration to afford a crude product, dissolve the crude product with ethyl acetate (5.3 L), wash with saturated ammonium chloride (3.5 L) solution, and concentrate the organic phase to dryness under reduced pressure to afford a 430 g of orange solid with a purity of 88.7% and a yield of 74.3%.
MS m/z: 1099.6 [M+H]⁺;
¹H NMR (400 MHz, DMSO-d6) δ 9.81 (s, 1H), 8.60 (d, J = 19.6 Hz, 2H), 8.42 (d, J = 3.8 Hz, 2H), 8.27 (dd, J = 11.2, 6.1 Hz, 5H), 7.49 (dd, J = 8.2, 4.0 Hz, 2H), 7.27 - 7.18 (m, 3H), 7.18 (s, 1H), 7.17 - 7.03 (m, 3H), 5.00 (dq, J = 26.8, 9.0 Hz, 5H), 3.87 (d, J = 4.9 Hz, 6H), 3.73 (t, J = 7.0 Hz, 2H), 3.22 (s, 2H), 2.87 (d, J = 16.3 Hz, 6H), 2.74 (dt, J = 11.6, 6.8 Hz, 4H), 2.30 (s, 3H), 2.19 (s, 4H).

### Preparation of compound 1

Add **SM-4** (430 g, 1.0 eq.) to the four-necked flask, add tetrahydrofuran (5.7 L) and water (2.9 L), start stirring, add sodium dithionite (511 g, 6.0 eq.) in batches, heat to 35°C-45 °C, allow to react for 3 hours while holding this temperature, monitor until the raw material reaction is complete, add concentrated hydrochloric acid (1430 ml, 44.0 eq.), allow to react overnight while hold the temperature at 45°C, monitor the liquid phase until the intermediate state is completely converted into the product, stop heating, add the system to the sodium hydroxide solution and, adjust the pH to 7-8 with saturated sodium bicarbonate solution, add ethyl acetate (4.3 L) for extraction, and concentrate the organic phase to dryness under reduced pressure to afford a 419 g of yellow solid (yield of 100%) with a liquid phase purity of 80.52%.
MS m/z: 1070.5[M+H]⁺;
¹H NMR (400 MHz, DMSO-d6) δ 8.62 (s, 1H), 8.44 (s, 1H), 8.37 - 8.21 (m, 5H), 7.49 (d, J = 8.1 Hz, 2H), 7.27 - 7.08 (m, 6H), 4.97 (q, J = 9.1 Hz, 2H), 4.85 (q, J = 9.2 Hz, 2H), 3.87 (d, J = 5.8 Hz, 6H), 3.11 (t, J = 6.6 Hz, 2H), 2.93 (s, 5H), 2.84 (s, 3H), 2.75 (d, J = 19.3 Hz, 6H), 2.62 (s, 3H), 2.35 (d, J = 18.8 Hz, 6H), 1.15 (t, J = 7.3 Hz, 13H).

### Example 2

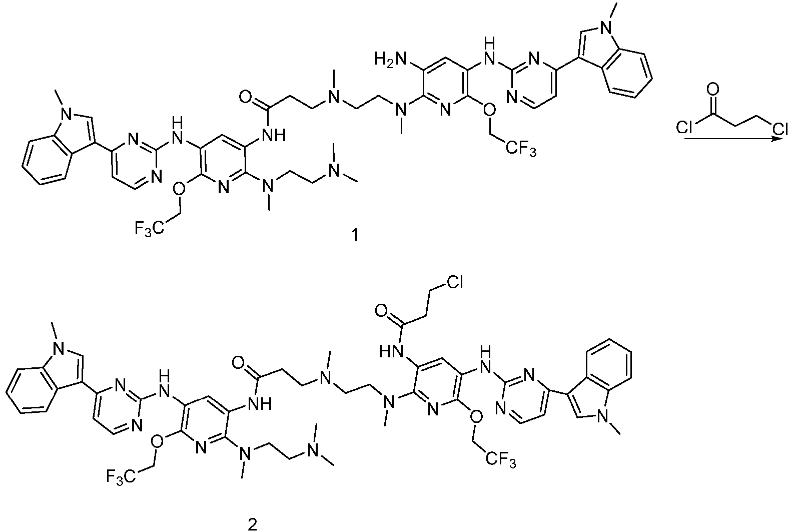

### Preparation of compound 2

Add a compound 1 (419 g, 1.0 eq.) to a four-necked flask, add dichloromethane (4.2 L) and triethylamine (80 g, 2.0 eq.), cool down to -40°C, slowly add a solution of 3-chloropropionyl chloride (75 g, 1.5 eq.) in methylene chloride (2 L) dropwise, monitor until the raw material reaction is complete, add a saturated sodium bicarbonate solution (4 L), stir and allow to settle for layering, separate the organic phase, extract a water phase with methylene chloride (1 L), combine organic phases, and concentrate under reduced pressure to dryness to afford 453 g of brown oil.

MS m/z: 1159.4[M+H]⁺.

### Example 3

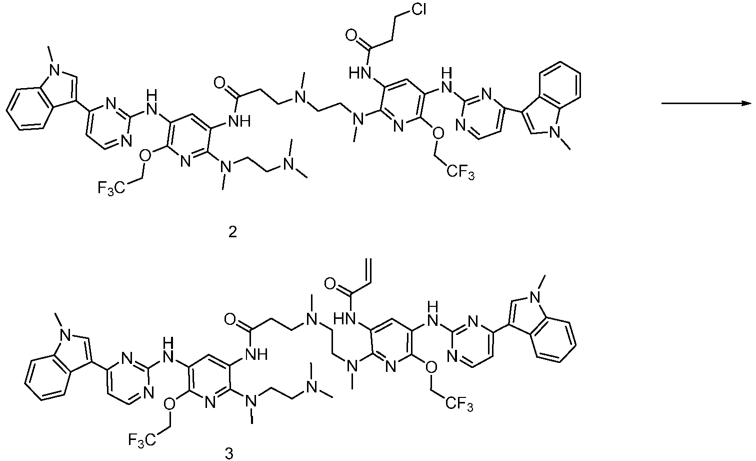

### Preparation of compound 3

Add a compound 2 (453 g, 1.0 eq.) to a four-necked flask, add acetonitrile (4.5 L), add triethylamine (198 g, 5.0 eq.), heat to 80°C, monitor until the raw material reaction is complete, cool down, carry out suction filtration, dry the crude product, perform column chromatography to afford a 133g of sample, add acetonitrile (1.3 L) and isopropyl ether (1.3 L) to the column product, slurry at 70°C, cool down, filter and drain, and dry to afford a 110g of pure product with a purity 98.5%.
MS m/z: 1123.6[M+H]⁺;
¹H NMR (400 MHz, DMSO-d6) δ 9.83 (s, 1H), 9.66 (s, 1H), 8.61 (s, 1H), 8.42 (d, J = 5.2 Hz, 2H), 8.25 (dq, J = 11.7, 5.2 Hz, 4H), 8.08 (d, J = 3.3 Hz, 2H), 7.49 (d, J = 8.1 Hz, 2H), 7.26-7.09 (m, 7H), 6.23 (dd, J = 17.1, 2.1 Hz, 1H), 5.71 (dd, J = 10.0, 2.1 Hz, 1H), 4.97 (qd, J = 9.0, 3.6 Hz, 5H), 3.87 (d, J = 2.1 Hz, 6H), 3.28-3.16 (m, 5H), 2.85 (d, J = 11.3 Hz, 7H), 2.74 (t, J = 7.1 Hz, 2H), 2.61 (t, J = 7.0 Hz, 2H), 2.43 (t, J = 6.7 Hz, 2H), 2.26 (s, 3H), 2.19 (s, 6H).

### Example 4

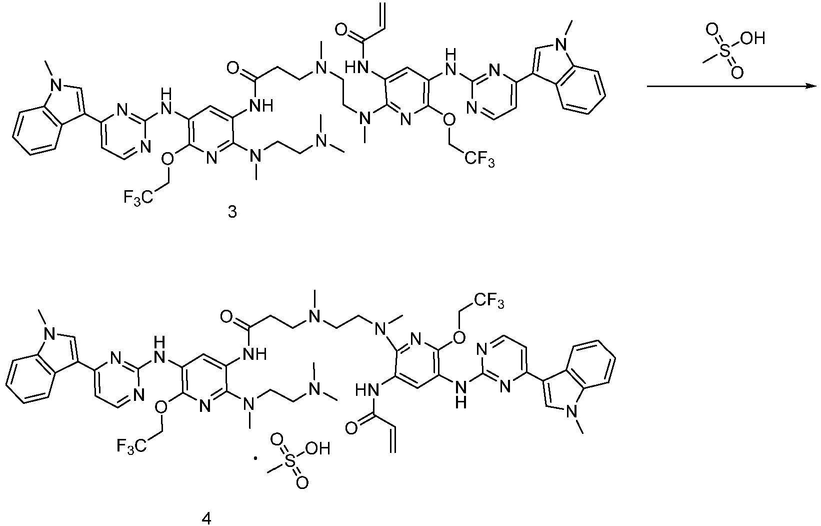

### Preparation of compound 4

Add a compound 3 (43.57 g, 1.0 eq.) to a four-necked flask, add dichloromethane (436 mL), stir at room temperature, slowly add a solution of methanesulfonic acid (3.54 g, 0.99 eq.) in dichloromethane (174 mL) dropwise, concentrate the reaction solution to dryness under reduced pressure, add the afforded solid to ethyl acetate (871 mL), slurry at 60°C-65°C, cool down, carry out suction filtration to afford a wet product, and then dry under vacuum at 35°C-40°C to afford 39 g of yellow solid powder with a purity of 98.7%.
MS m/z: 1123.5 [M+H]⁺;
¹H NMR (400 MHz, DMSO-d6) δ 9.73 (br, 2H), 8.52 (s, 1H), 8.47 (s, 1H), 8.39 (d, J =8.8 Hz, 2H), 8.29-8.26 (m, 4H), 8.16 (br, 1H), 8.13 (br, 1H), 7.49 (d, J = 8.1 Hz, 2H), 7.25 - 7.13 (m, 6H), 6.57 (dd, J = 16.8, 10.3 Hz, 1H), 6.28 (d, J = 10.7, 17.0 Hz, 1H),5.75 (d, J = 10.7, 17.0 Hz, 1H), 4.99 (q, J = 8.9Hz, 4H), 3.86 (s, 6H), 3.56 (s, 2H), 3.40(br, 1H), 3.16 (br, 2H), 2.98 (br, 7H), 2.85 (S, 3H), 2.83 (S, 3H), 2.74 (br, 9H), 2.42 (s, 3H).

### Test Example 1: Proliferation inhibitory activity against BalF3 EGFR-Del19/T790M/C797S cells

The proliferation inhibitory activity of the compounds in vitro against the BalF3 EGFR-Del19/T790M/C797S cells from the murine pro B cell line BalF3 that stably express EGFR proteins with Del19/T790M/C797S triple mutations, is determined by the PrestoBlue method.

Cell source: BalF3 EGFR-Del19/T790M/C797S cells are purchased from Crown Bioscience (Beijing) Co., Ltd.

BalF3 EGFR-Del19/T790M/C797S cells are cultured in RPMI1640 complete medium containing 10% fetal calf serum. Take BalF3 EGFR-Del19/T790M/C797S cells in the logarithmic growth phase, inoculate them in a 96-well plate at a cell density of 5000 cells/135 µl complete culture medium/well, and place them at a constant temperature incubator containing 5% CO₂ at 37°C for culturing for 24 hours. Each compound is dissolved in dimethyl sulfoxide (DMSO) in advance to prepare a 10 mM stock solution, and then diluted with DMSO and a complete culture medium in sequence. Take out the 96-well plate in which cells are inoculated, and add 15 µl of compounds of different concentrations to each well so that the final concentration is 2500 nM, 625 nM, 156.25 nM, 39.06 nM, 9.77 nM, 2.44 nM, 0.61 nM, 0.15 nM, 0.04 nM, 0.01 nM, set three duplicate wells for each compound concentration, and set a negative control group (containing a cell culture medium control group) and a blank control group (a cell-free culture medium control group), where the concentration of DMSO in each well is 0.5%. Continue to culture for 72 hours in the constant temperature incubator containing 5% CO₂ at 37°C.

Take out the 96-well cell culture plate from the CO₂ constant temperature incubator, add 15 µl of Invitrogen^{™} PrestoBlue^{™} HS cell viability detection reagent (Cat. No.: P50201) to each well, and continue to incubate for 3 hours in the constant temperature incubator containing 5% CO₂ at 37°C. Take out the 96-well cell culture plate and measure the fluorescence at the 560 nm excitation wavelength and 590 nm emission wavelength of a microplate reader. Calculate the cell inhibition rate for each concentration of the compound according to the following formula. Cell inhibition rate = (RFU72 hours negative control group-RFUadministration 72 hours compound group)/(RFU72 hours negative control group-RFU72 hours blank control group)] × 100% (RFU is the relative fluorescence intensity).

Data were analyzed using GraphPad Prism 8.3 software, and nonlinear S-curve regression was used to fit the data to obtain a dose-effect curve, from which the IC₅₀ value was calculated. The results are as shown in Table 1.

**Table 1**

| **Compound** | **BalF3 EGFR-De119/T790M/C797S IC₅₀ (µM)** |
|---|---|
| Compound 1 | 1.863 |
| Compound 4 | 0.293 |

The test results show that the compound of the present invention has good proliferation inhibitory activity against BalF3 EGFR-Del19/T790M/C797S cells.

## Claims

1. An indole bipyrimidine compound as shown in formula I or a pharmaceutically acceptable salt thereof, **characterized in that** 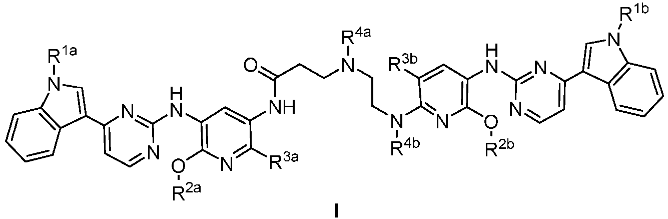
wherein, R^{1a} and R^{1b} are each independently H or C₁₋₄ alkyl;
R^{2a} and R^{2b} are each independently C₁₋₄ alkyl, and C₁₋₄ alkyl substituted by one or more R^{1-a}; when there is a plurality of substituents, they are the same or different;
R^{1-a} is independently halogen, -O-(C₁₋₄ alkyl), -N(R^{a1})(R^{a2}) or -C(=O)-(C₁₋₄ alkyl);
R^{a1} and R^{a2} are each independently H or C₁₋₄ alkyl;
R^{3a} and R^{3b} are each independently -N(R^{b1})(R^{b2});
R^{b1} and R^{b2} are each independently H, C₁₋₄ alkyl, -C₁₋₄ alkylene-N(R^{c1})(R^{c2}) or -C(=O)-R^{c3};
R^{c1}, R^{c2} and R^{c3} are each independently H, C₁₋₄ alkyl, C₁₋₄ alkyl substituted by one or more halogens, C₂₋₄ alkenyl, or C₂₋₄ alkenyl substituted by one or more halogens; and
R^{4a} and R^{4b} are each independently H or C₁₋₄ alkyl.

2. The indole bipyrimidine compound, as shown in formula I, of claim 1, or a pharmaceutically acceptable salt thereof, **characterized in that** the indole bipyrimidine compound as shown in formula I or a pharmaceutically acceptable salt thereof satisfies one or more of the following conditions:
(1) when R^{1a} and R^{1b} are each independently C₁₋₄ alkyl, said C₁₋₄ alkyl is methyl, ethyl, n-propyl, isopropyl, n-butyl, isobutyl, sec-butyl or tert-butyl;
(2) when R^{2a} and R^{2b} are each independently C₁₋₄ alkyl or C₁₋₄ alkyl substituted by one or more R^{1-a}, C₁₋₄ alkyl in said C₁₋₄ alkyl and said C₁₋₄ alkyl substituted by one or more R^{1-a} are methyl, ethyl, n-propyl, isopropyl, n-butyl, isobutyl, sec-butyl or tert-butyl;
(3) when there is a plurality of R^{1-a}, the number thereof is 2, 3, 4 or 5;
(4) when R^{1-a} is independently halogen, the halogen is independently fluoro, chloro, bromo or iodo;
(5) when R^{1-a} is independently -O-(C₁₋₄ alkyl) or -C(=O)-(C₁₋₄ alkyl), C₁₋₄ alkyl in the -O-(C₁₋₄ alkyl) and the -C(=O)-(C₁₋₄ alkyl) is methyl, ethyl, n-propyl, isopropyl, n-butyl, isobutyl, sec-butyl or tert-butyl;
(6) when R^{a1} and R^{a2} are each independently C₁₋₄ alkyl, said C₁₋₄ alkyl is methyl, ethyl, n-propyl, isopropyl, n-butyl, isobutyl, sec-butyl or tert-butyl;
(7) when R^{b1} and R^{b2} are each independently C₁₋₄ alkyl, the C₁₋₄ alkyl is methyl, ethyl, n-propyl, isopropyl, n-butyl, isobutyl, sec-butyl or tert-butyl;
(8) when R^{b1} and R^{b2} are each independently -C₁₋₄ alkylene-N(R^{c1})(R^{c2}), the -C₁₋₄ alkylene is -CH₂-, -CH₂CH₂-, -CH(CH₃)-, -CH(CH₃)CH₂- or -C(CH₃)₂-;
(9) when R^{c1}, R^{c2}, R^{c3}, R^{4a} and R^{4b} are each independently C₁₋₄ alkyl, said C₁₋₄ alkyl is methyl, ethyl, n-propyl, isopropyl, n-butyl, isobutyl, sec-butyl or tert-butyl;
(10) when R^{c1}, R^{c2} and R^{c3} are each independently C₁₋₄ alkyl substituted by one or more halogens, the C₁₋₄ alkyl is methyl, ethyl, n-propyl, isopropyl, n-butyl, isobutyl, sec-butyl or tert-butyl;
(11) when R^{c1}, R^{c2} and R^{c3} are each independently C₂₋₄ alkenyl or C₂₋₄ alkenyl substituted by one or more halogens, C₂₋₄ alkenyl in said C₂₋₄ alkenyl and said C₂₋₄ alkenyl substituted by one or more halogens is ethenyl, propenyl or allyl;
(12) when R^{2a} and R^{2b} are each independently C₁₋₄ alkyl substituted by one or more R^{1-a} and R^{1-a} is halogen, the C₁₋₄ alkyl substituted by R^{1-a} is trifluoromethyl or -CH₂CF₃;
(13) when R^{c1}, R^{c2} and R^{c3} are each independently C₁₋₄ alkyl substituted by one or more halogens, the C₁₋₄ alkyl substituted by halogens is -CH₂CH₂Cl or -CH₂CF₃; and
(14) one of R^{b1} and R^{b2} is C₁₋₄ alkyl, and the other is -C₁₋₄ alkylene-N(R^{c1})(R^{c2}); or one of R^{b1} and R^{b2} is H, and the other is H or -C(=O)-R^{c3}.

3. The indole bipyrimidine compound, as shown in formula I, of claim 1, or a pharmaceutically acceptable salt thereof, **characterized in that** the indole bipyrimidine compound as shown in formula I or a pharmaceutically acceptable salt thereof satisfies one or more of the following conditions:
(1) when R^{1a} and R^{1b} are each independently C₁₋₄ alkyl, the C₁₋₄ alkyl is methyl or ethyl;
(2) when R^{2a} and R^{2b} are each independently C₁₋₄ alkyl or C₁₋₄ alkyl substituted by one or more R^{1-a}, C₁₋₄ alkyl in said C₁₋₄ alkyl and said C₁₋₄ alkyl substituted by one or more R^{1-a} is methyl or ethyl;
(3) when there is a plurality of R^{1-a}, the number thereof is 2 or 3;
(4) when R^{1-a} is independently halogen, the halogen is independently fluoro or chloro;
(5) when R^{1-a} is independently -O-(C₁₋₄ alkyl) or -C(=O)-(C₁₋₄ alkyl), C₁₋₄ alkyl in said - O-(C₁₋₄ alkyl) and said -C(=O)-(C₁₋₄ alkyl) is methyl or ethyl;
(6) when R^{a1} and R^{a2} are each independently C₁₋₄ alkyl, said C₁₋₄ alkyl is methyl or ethyl;
(7) when R^{b1} and R^{b2} are each independently C₁₋₄ alkyl, the C₁₋₄ alkyl is methyl or ethyl;
(8) when R^{b1} and R^{b2} are each independently -C₁₋₄ alkylene-N(R^{c1})(R^{c2}), the C₁₋₄ alkylene is -CH₂- or -CH₂CH₂-;
(9) when R^{c1}, R^{c2}, R^{c3}, R^{4a} and R^{4b} are each independently C₁₋₄ alkyl, said C₁₋₄ alkyl is methyl or ethyl;
(10) when R^{c1}, R^{c2} and R^{c3} are each independently C₁₋₄ alkyl substituted by one or more halogens, the C₁₋₄ alkyl is methyl or ethyl;
(11) when R^{c1}, R^{c2} and R^{c3} are each independently C₂₋₄ alkenyl or C₂₋₄ alkenyl substituted by one or more halogens, C₂₋₄alkenyl in said C₂₋₄ alkenyl and said C₂₋₄ alkenyl substituted by one or more halogens is ethenyl;
(12) R^{b1} and R^{b2} are each independently selected from H, methyl, 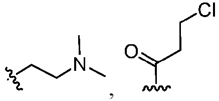 and 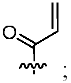 and
(13) one of R^{3a} and R^{3b} is 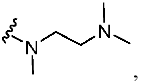 and the other is NH₂,

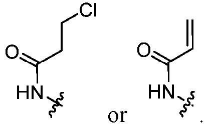

4. The indole bipyrimidine compound, as shown in formula I, of any one of claims 1-3, or a pharmaceutically acceptable salt thereof, **characterized in that** the indole bipyrimidine compound as shown in formula I is the following compound: 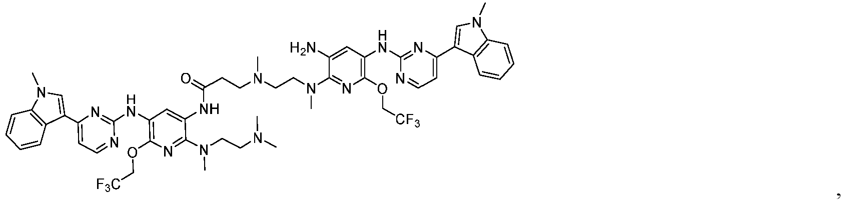 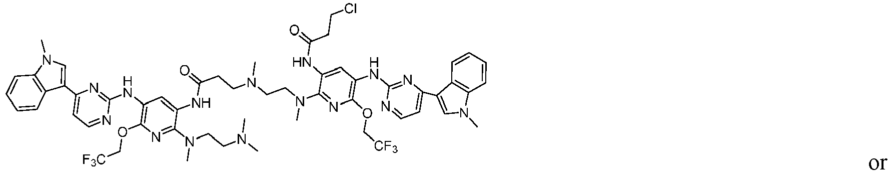 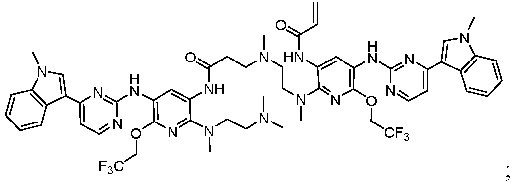 or a pharmaceutically acceptable salt of the indole bipyrimidine compound as shown in formula I is the following compound:

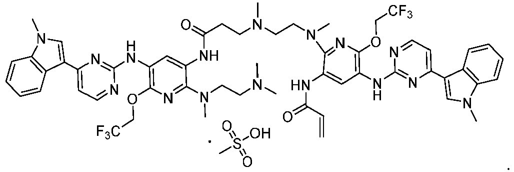

5. A preparation method for an indole bipyrimidine compound, as shown in formula I, of any one of claims 1-4, **characterized in that** it can be of the following routes:
route I
when R^{3b} is -NH₂, the corresponding indole bipyrimidine compound as shown in formula I is as shown in formula I-1, which is prepared by the following method: 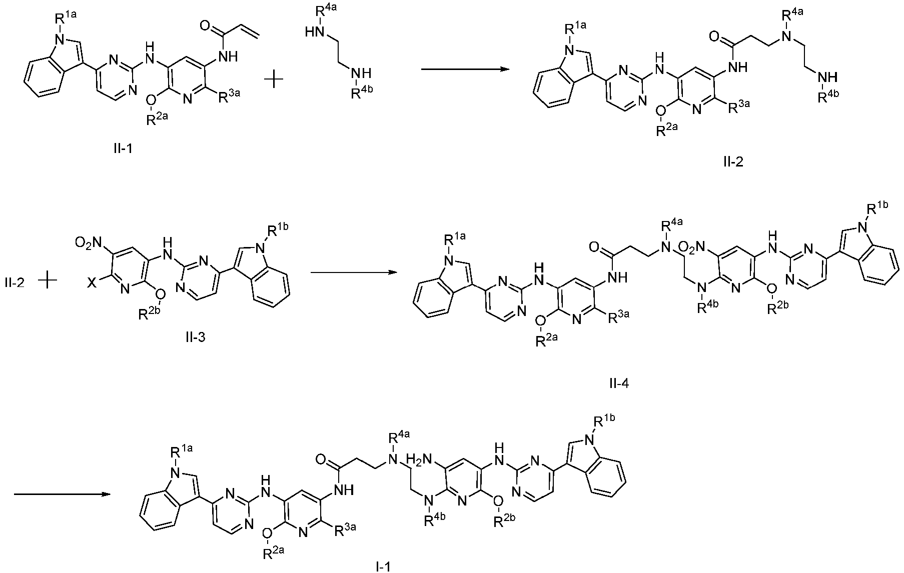
wherein, R^{1a}, R^{1b}, R^{2a}, R^{2b}, R^{3a}, R^{4a} and R^{4b} are as defined in any one of claims 1-4; X is a leaving group;
step 1: in a solvent and in the presence of a base, a compound as shown in formula II-1 and a compound as shown in formula (R^{4a}H)NCH₂CH₂N(HR^{4b}) are subjected to an addition reaction as shown to afford a compound as shown in formula II-2;
step 2: in a solvent and in the presence of a base, the compound as shown in formula II-2 and a compound as shown in formula II-3 or a salt thereof are subjected to a substitution reaction as shown to afford a compound as shown in formula II-4; and
step 3: in a solvent and in the presence of a reducing agent, the compound as shown in formula II-4 is subjected to a nitro reduction reaction as shown, and optionally, an acid is added to promote the completion of the reduction reaction, so as to afford the compound as shown in formula I-1;
route II
when R^{3b} is -NH-C(=O)-R^{c3}, the corresponding indole bipyrimidine compound as shown in formula I is as shown in formula I-2, which is prepared by the following method: 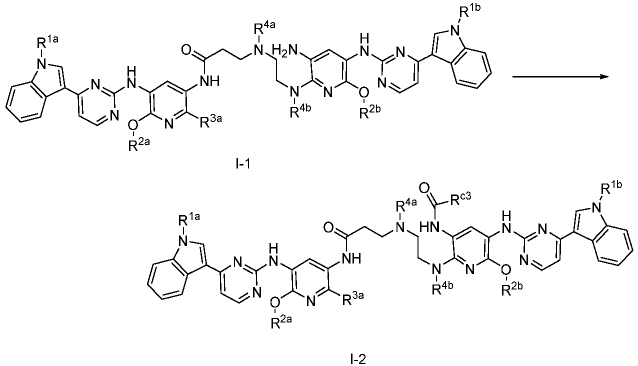
wherein, R^{1a}, R^{1b}, R^{2a}, R^{2b}, R^{3a}, R^{c3}, R^{4a} and R^{4b} are as defined in any one of claims 1-4;
in a solvent and in the presence of a base, the compound as shown in formula I-1 and a compound as shown in formula Cl-C(=O)-R^{c3} are subjected to a substitution reaction as shown to afford a compound as shown in formula I-2;
route III
when R^{3b} is -NH-C(=O)-CH=CH₂, the corresponding indole bipyrimidine compound as shown in formula I is as shown in formula I-2b, which is prepared by the following method: 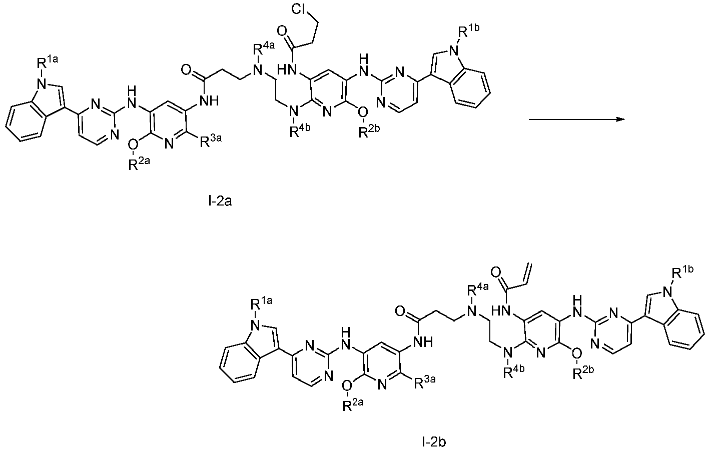
wherein, R^{1a}, R^{1b}, R^{2a}, R^{2b}, R^{3a}, R^{4a} and R^{4b} are as defined in any one of claims 1-4;
in a solvent and in the presence of a base, a compound as shown in formula I-2a is subjected to an elimination reaction as shown to afford a compound as shown in formula I-2b.

6. The preparation method for an indole bipyrimidine compound, as shown in formula I, of claim 5, **characterized in that**
in the route I, one or more of the following conditions are satisfied:
(1) X is halogen, such as Cl;
(2) in step 1, said base is an organic base, such as triethylamine, N,N-diisopropylethylamine or N,N'-dimethylethylenediamine;
(3) in step 1, said solvent is tetrahydrofuran;
(4) in step 1, the reaction is heating until the system refluxes;
(5) in step 1, a molar ratio of said compound as shown in formula II-1 to the compound as shown in formula (R^{4a}H)NCH₂CH₂N(HR^{4b}) is 1:5;
(6) in step 1, a molar ratio of said compound as shown in formula II-1 to the base is 1:2;
(7) in step 1, a mass-to-volume ratio of the compound as shown in formula II-1 to the solvent is 0.1 Kg/L to 0.2 Kg/L, such as 0.1 Kg/L;
(8) in step 2, said base is an inorganic base, such as potassium carbonate;
(9) in step 2, said solvent is DMF or DMSO;
(10) in step 2, the temperature of the reaction is 30°C to 80°C, such as 65°C;
(11) in step 2, said salt of the compound as shown in formula II-3 is p-toluenesulfonate of said compound;
(12) in step 2, a molar ratio of the compound as shown in formula II-2 to the compound as shown in formula II-3 or a salt thereof is 0.9:1 to 1:0.9, such as 1:0.95;
(13) in step 2, a molar ratio of the compound as shown in formula II-2 to the base is 1:3;
(14) in step 2, a mass-to-volume ratio of the compound as shown in formula II-2 to the solvent is 0.1 Kg/L to 0.2 Kg/L, such as 0.144 Kg/L;
(15) in step 3, said reducing agent is sodium dithionite, iron powder, zinc powder or H₂, wherein reduction of the compound as shown in formula II-4 with H₂ is carried out in the presence of a catalyst such as Pd/C and Pd(OH)₂/C;
(16) in step 3, said solvent is tetrahydrofuran and water, or is ethanol and water;
(17) in step 3, the temperature of the reaction is 25°C to 50°C, such as 35°C to 45°C;
(18) in step 3, a molar ratio of the compound as shown in formula II-4 to said reducing agent is 1:6;
(19) in step 3, a mass-to-volume ratio of the compound as shown in formula II-4 to the solvent is 0.05 Kg/L to 0.1 Kg/L, such as 0.05 Kg/L;
(20) in step 3, said acid is a concentrated hydrochloric acid; and
(21) in step 3, a molar ratio of the compound as shown in formula II-4 to said acid is 1:44;
in the route II, one or more of the following conditions are satisfied:
(1) said base is an organic base, such as triethylamine or N,N-diisopropylethylamine;
(2) said solvent is dichloromethane or tetrahydrofuran;
(3) the temperature of the reaction is -50°C to 10°C, such as -40°C;
(4) a molar ratio of the compound as shown in formula I-1 to the compound as shown in formula Cl-C(=O)-R^{c3} is 1:1.5;
(5) a molar ratio of the compound as shown in formula I-1 to the base is 1:2; and
(6) a mass-to-volume ratio of the compound as shown in formula I-1 to the solvent is 0.05 Kg/L to 0.15 Kg/L, such as 0.1 Kg/L;
in the route III, one or more of the following conditions are satisfied:
(1) said base is an organic base, such as triethylamine or N,N-diisopropylethylamine;
(2) said solvent is acetonitrile or DMSO;
(3) the temperature of the reaction is 50°C to 100°C, such as 80°C;
(4) a molar ratio of said compound as shown in formula I-2a to the base is 1:5; and
(5) a mass-to-volume ratio of the compound as shown in formula I-2a to the solvent is 0.05 Kg/L to 0.15 Kg/L, such as 0.1 Kg/L.

7. A compound as shown below,

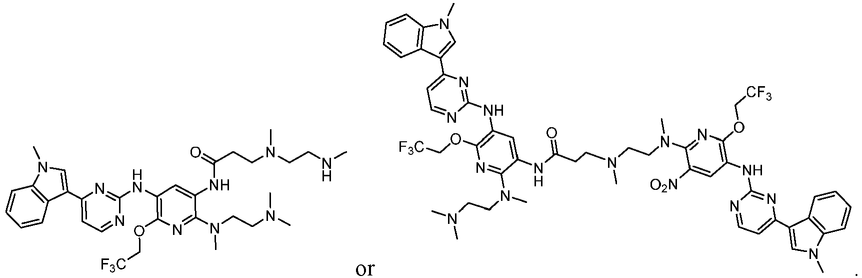

8. A pharmaceutical composition, **characterized in** comprising a therapeutically effective amount of substance A and pharmaceutical auxiliary materials; and said substance A is the indole bipyrimidine compound, as shown in formula I, of any one of claims 1-4 or a pharmaceutically acceptable salt thereof.

9. Use of a substance A in the preparation of a EGFR inhibitor, **characterized in that** said substance A is the indole bipyrimidine compound, as shown in formula I, of any one of claims 1-4 or a pharmaceutically acceptable salt thereof.

10. Use of a substance A in the preparation of a medicament, **characterized in that** said medicament is for use in the treatment and/or prevention of a disease mediated by EGFR, or said medicament is for use in the treatment and/or prevention of cancer; said substance A is the indole bipyrimidine compound, as shown in formula I, of any one of claims 1-4 or a pharmaceutically acceptable salt thereof; and said substance A is at a therapeutically effective amount.

11. The use of claim 10, **characterized in that** said disease mediated by EGFR is a disease resistant to first-, second- and third- generation EGFR inhibitors; said first-, second- and third-generation EGFR inhibitors can be selected from gefitinib, erlotinib, icotinib, afatinib, dacomitinib and osimertinib;
and/or said disease mediated by EGFR is a disease mediated by an EGFR Del19/T790M/C797S mutation;
and/or said disease mediated by EGFR is cancer; said cancer can be selected from one or more of colon cancer, pancreatic cancer, breast cancer, prostate cancer, lung cancer, brain cancer, ovarian cancer, cervical cancer, testicular cancer, renal cancer, cancer of the head or neck, bone cancer, skin cancer, rectal cancer, liver cancer, colorectal cancer, non-small cell lung cancer, small cell lung cancer, adenocarcinoma of the lung, squamous carcinoma of the lung, esophageal cancer, gastric cancer, thyroid cancer, bladder cancer, lymphoma, glioma, glioblastoma, gastrointestinal stromal tumor, cholangiocarcinoma, endometrial cancer, multiple myeloma, leukemia and melanoma;
and/or said cancer is selected from one or more of colon cancer, pancreatic cancer, breast cancer, prostate cancer, lung cancer, brain cancer, ovarian cancer, cervical cancer, testicular cancer, renal cancer, cancer of the head or neck, bone cancer, skin cancer, rectal cancer, liver cancer, colorectal cancer, non-small cell lung cancer, small cell lung cancer, adenocarcinoma of the lung, squamous carcinoma of the lung, esophageal cancer, gastric cancer, thyroid cancer, bladder cancer, lymphoma, glioma, glioblastoma, gastrointestinal stromal tumor, cholangiocarcinoma, endometrial cancer, multiple myeloma, leukemia and melanoma.
